# EUROPEAN PATENT SPECIFICATION

(11) **EP 0 827 514 B1**
(45) Date of publication and mention of the grant of the patent: **11.08.1999**
(21) Application number: 96916030.8
(22) Date of filing: 08.05.1996
(51) Int. Cl.: C08B 37/08, A61K 47/48, A61K 33/24

(54) **HEAVY METAL SALTS OF SUCCINIC ACID HEMIESTERS WITH HYALURONIC ACID, OR HYALURONIC ACID ESTERS, A PROCESS FOR THEIR PREPARATION, AND RELATIVE PHARMACEUTICAL COMPOSITIONS**
SCHWERMETALLSALZE VON BERNSTEINSÄUREHALBESTER MIT HYALURONSÄURE ODER HYALURONSÄUREESTER, VERFAHREN ZU IHRER HERSTELLUNG UND VERBUNDENE PHARMAZEUTISCHE ZUSAMMENSETZUNGEN
SELS DE METAUX LOURDS D'HEMIESTERS D'ACIDE SUCCINIQUE AVEC DE L'ACIDE HYALURONIQUE OU DES ESTERS D'ACIDE HYALURONIQUE, PROCEDE DE PREPARATION ET COMPOSITIONS PHARMACEUTIQUES ASSOCIEES

(30) Priority: 10.05.1995 IT PD950090
(43) Date of publication of application: 11.03.1998
(73) Proprietor: FIDIA ADVANCED BIOPOLYMERS S.R.L., 72100 Brindisi (IT)
(72) Inventor: KHAN, Riaz, Sonning, Berkshire RG4 6TA (GB); KONOWICZ, A., Paul, Surrey GU9 OR2 (GB); FLAIBANI, Antonella, I-33100 Udine (IT); GOMBAC, Valentina, I-34125 Trieste (IT)
(74) Representative: Passini, Angelo
(86) International application number: EP9601979
(87) International publication number: WO9635720

(56) References cited:
- EP-A- 0 066 283
- EP-A- 0 314 835
- US-A- 4 746 504
- US-A- 4 851 521
- DATABASE WPI Week 7917 Derwent Publications Ltd., London, GB; AN 32582B XP002013867 & JP,A,54 036 388 (SUMITOMO ELEC IND KK) , 17 March 1979

## Description

### FIELD OF THE INVENTION

The present invention relates to succinic acid hemiesters with hyaluronic acid or with hyaluronic acid partial or total esters, and heavy metal salts of said succinic hemiesters with hyaluronic acid or hyaluronic acid total or partial esters , a process for their preparation and pharmaceutical compositions containing these salts as active ingredients.

### BAGKGROUND OF THE INVENTION

Hyaluronic acid is a polysaccharide whose chain is constituted by alternating units of 1,4-β-D-glucuronic acid and 1,3-β-N-acetyl-D-glucosamine. Hyaluronic acid is a fundamental component of the connective tissue of animals, being present, for example, in the skin and cartilage. It is also found in high concentrations in the umbilical cord, in the synovial fluid and vitreous humor of the eye. Currently, the preferred source of hyaluronic acid is by extraction from cockscombs, even though the production of hyaluronic acid from Streptococcus cultures is becoming increasingly widespread (T. J. Lieselang. Survey of Ophthalmology 34,268-293, 1990).

As hyaluronic acid is a fundamental component of the connective tissue, it is biocompatible, bioadsorbable and not immunogenic. It therefore plays a key role in many biological functions, such as tissue hydration, the organization of proteoglycans in the cartilage, tissue repair, embryonic development and lubrication and protection of joint cartilage. This polysaccharide is commonly used in the treatment of some joint diseases, such as rheumatoid arthritis. It is also used in what is known as microviscosurgery, and in particular, in surgery to the eye. In this application, the biocompatibility and rheological characteristics of concentrated solutions of high-molecular-weight hyaluronic acid are exploited.

In cases of inflammation of the joints, hyaluronic acid is degraded by superoxide radicals (Greenwald R. A. et al., Inflammation, 10, 15-30, 1986). This degradation determines a notable reduction in the rheological and viscoelastic characteristics of the synovial fluid, markedly reducing the lubricant and protective effect which hyaluronic acid has on the cartilage. It has been hypothesized that the superoxide dismutase enzyme constitutes the main defense against damage caused by the superoxide radical which is produced in the course of inflammatory processes. Copper and zinc are components of the superoxide dismutase enzyme, the function of which seems to be to protect cells from the toxic effects of endogenous superoxide radicals.

Rheumatoid arthritis has been associated with zinc deficiencies and an antiinflammatory activity has been hypothesized for zinc itself (A. Frigo et al., "Copper and Zinc in Inflammation", Inflammation and drug therapy series, Vol. IV. Kluwer Academic Publishers, pp. 133-142, 1989). Treatment with zinc sulfate has proved to be efficacious in controlling joint disorders caused by arthritis in patients affected by psoriasis.

In the same way, alterations in copper concentrations have been observed in patients affected by inflammatory diseases in the joints (C. W. Denko, "Copper and Zinc in Inflammation, Inflammation and drug therapy series, Vol. IV, Kluwer Academic Publishers, pp. 1-5, 1989). Copper-based compounds have been used to treat rheumatoid arthritis and their activity is attributed to copper ions.

Gold salts are also used as drugs to treat arthritis, together with known antiinflammatory products of a steroid and non-steroid type (US 4,746,504).

Many silver salts, such as silver fluoride, silver iodide, silver lactate, have been used as antibacterial agents for topical use. Their antimicrobial activity is due to the action of the Ag+ ions. Heavy metal salts of hyaluronic acid are therefore already known to the state of the art, such as silver, gold, cerium and tungsten. The reaction between a sodium hyaluronate aqueous solution and a silver nitrate solution gives the silver salt of hyaluronic acid. Pharmaceutical preparations containing all these compounds are used to advantage for the treatment of burns, wounds and some ophthalmic infections such as gonococcus-induced conjunctivitis (A. Nimrod and B. Greenman, US Patent No. 4,746,504, May 24, 1988).

However, neither hyaluronic acid nor hyaluronic acid partial or total ester derivatives wherein one or more hydroxy functions of its 1,4 β-D-glucuronic acid and 1,3-β-N-acetyl-D-glucosamine alternating repeating units esterified with a carboxyl group of succinic acid to form the succinic hemiester of hyaluronic acid or hyaluronic acid total or partial esters are known to the state of the art.

### SUMMARY OF THE INVENTION

The present invention concerns a succinic hemiester of hyaluronic acid or of a hyaluronic acid total or partial ester and its inorganic salt with a heavy metal.

In particular the succinic acid hemiester with hyaluronic acid, or with a hyaluronic acid total or partial ester is characterized by having the following repeating unit (I): 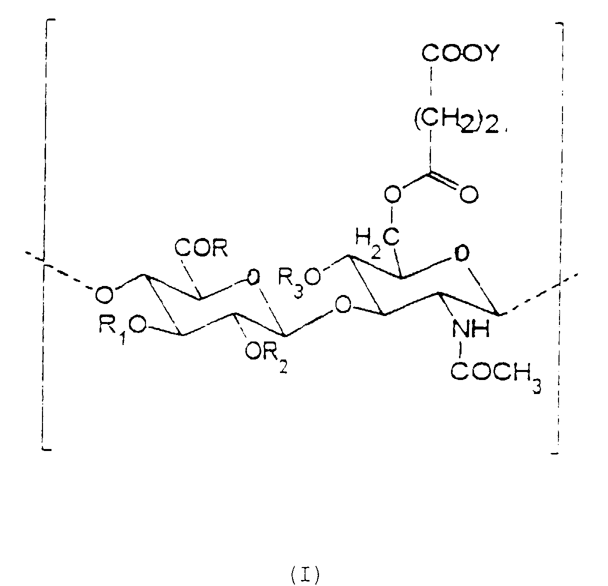 wherein R₁, R₂ and R₃ equal or different from each other are H or CO-(CH₂)₂-COOY, wherein Y is H, R is OH, or an alcoholic residue.

The hyaluronic acid esters contemplated for preparing the succinic acid hemiester are the total or partial ester with alcohol of the aliphatic or cycloaliphatic series, which do not themselves possess a notable pharmacological action disclosed in USP 4,851,521, which we incorporate herewith by reference.

The heavy metal salt of the succinic acid hemiester with hyaluronic acid or with a hyaluronic acid total or partial ester are in particular characterized by having the following repeating unit (II): 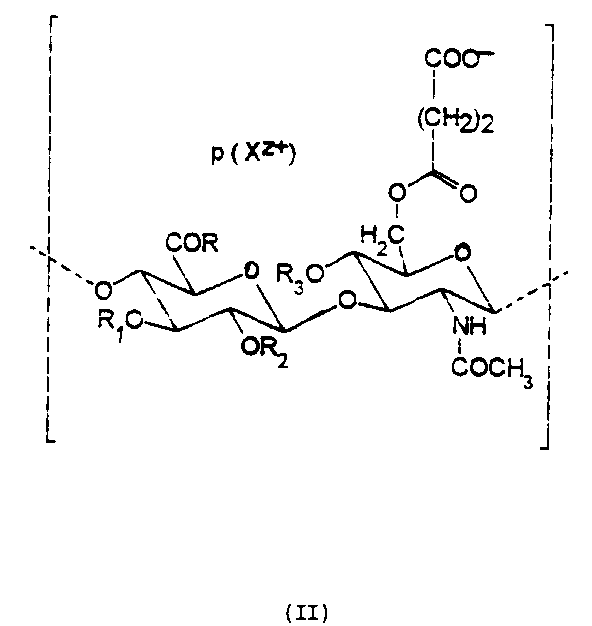 wherein R₁, R₂ and R₃ equal or different from each other are H or CO-(CH₂)₂-COO⁻, R is O⁻, or an alcoholic residue, (X^{z+}) is a cation of a heavy metal in which z is a number comprised between 1 and 6. p is an integer or a decimal number, comprised between 0.1 and 5 provided that p(X^{z+}) is equal to the number of anionic groups COO⁻ present in said repeating unit. The heavy metal salts according to the present invention are characterized by having a far greater negative charge density than the corresponding heavy metal salt of the starting hyaluronate . Indeed, the new substituting group, i.e. succinic acid, can bind, theoretically, to all the alcoholic functions of the repeating unit, giving a polysaccharide containing up to four succinic groups per repeating unit and therefore four more negative charges available for the formation of salts.

A further subject of the present invention relates to the process for preparing said succinic acid hemiester with hyaluronic acid or with a hyaluronic acid partial ester and the corresponding heavy metal salt.

This process in particular comprises the following steps:
a) converting the hyaluronic acid sodium salt into a salt selected from the group consisting of pyridinium, tetraalkylammonium. tetraarylammonium, tetraalkylphosphonium, tetraarylphosphonium salt, in the presence of water and an aprotic solvent,
b) treating the solution coming from step (a) with succinic anhydride in the presence of an organic base, as the catalyst, removing the pyridinium tetraalkylammonium, tetraarylammonium, tetraalkylphosphonium, or tetraarylphosphonium cation by dialysis, thereby obtaining the succinic acid hemiester having the repeating unit (I) provided that at least one of said repeating units (I) has R = OH, and optionally recovering the obtained product by freeze-drying.
c) treating the solution directly coming from the preceding step or an aqueous solution of the recovered solid product coming from the preceding step with an aqueous solution of an inorganic salt of the heavy metal, and recovering the product by filtration and vacuum drying.

In case of the preparation of the heavy metal salt with the succinate hemiester of the total ester of hyaluronic acid, the process according to the present invention contemplates the following steps:
b') treating the hyaluronic acid ester dissolved or suspended in a mixture of water and an aprotic solvent with succinic anhydride in the presence of an organic base, as the catalyst, thereby obtaining the succinic acid hemiester having the repeating units (I) wherein R is a residue of an alcohol, and optionally recovering the obtained product by freeze-drying,
c') treating the solution directly coming from the preceding step or an aqueous solution of the recovered solid product coming from the preceding step with an aqueous solution of an inorganic salt of the heavy metal, and recovering the product by filtration and vacuum drying.

The heavy metal salts of succinic acid hemiester with a hyaluronic acid or with a partial or total hyaluronic acid ester according to the present invention can be used to advantage as antimicrobial, antibacterial and disinfectant agents, for the treatment of wounds, burns and ophthalmia, or they can be incorporated in suitable pharmaceutical forms, optionally in association with one or more other pharmacologically active substances, having a similar therapeutic activity.

In addition they can be advantageously used as disinfectant agents, not only for the preparation of drugs, but also as active ingredients for the preparation of the so-called health care products, such as cosmetic creams and ointments, shave and after shave lotions and hair lotions etc. or biomaterials such as membranes non-woven tissues, gauzes, etc.

The heavy metal salts of succinyl monoesters of hyaluronic acid can be also advantageously used as antiinflammatory agents in the treatment of arthritis and inflammations affecting the joints optionally in association with other pharmaceutically active principles having a similar therapeutical activity.

### DETAILED DESCERPTION OF THE INVENTION

The term "heavy metal" encompasses any pharmaceutically active metal in the 4, 5 or 6 period of the periodic table.

The preferred heavy metal salts according to the present invention are those whose cation is: zinc, silver, copper, gold, cerium and tungsten salts of succinic derivatives of hyaluronic acid.

It has in fact been found that compared with the corresponding salts with hyaluronic acid or with hyaluronic acid partial esters these salts offer an advantage over the already-known products containing heavy metal salts, because the salts according to the present invention can bind a high number of heavy metal cations. Indeed. while hyaluronic acid can bind only one counter-ion per repeating unit, the salts according to the present invention bind at least twice as many counter-ions per repeating unit.

It is therefore advantageous to use these heavy metal salts with higher concentrations of metal for the therapeutic applications identified and described in the text, as this is the most active component in the preparation.

Hyaluronic acid or hyaluronic acid esters of any molecular weight can be used to prepare succinyl derivatives thereof. In the present invention, samples of hyaluronic acid with a molecular weight of between 30,000 and 760,000 Daltons were used, but this range is not critical for the purpose of the present invention.

Preferred succinic acid hemiesters of hyaluronic acid or hyaluronic acid esters are those having in the repeating unit (I) R₁ = R₂ = R₃ = H and the corresponding heavy metal salts wherein in the repeating unit (II) X is selected from the group consisting of: silver, gold, copper, zinc, z is comprised between 1 and 3 and p is comprised between 0.3 and 2.

Another class of preferred succinic acid hemiesters with hyaluronic acid or hyaluronic acid esters are those having at least one repeating unit (I) wherein R₁= R₃ = H and R₂ = CO-(CH₂)₂-COOY and at least one repeating unit (I), wherein R₂ = R₃ = H, and R₁ =CO-(CH₂)₂-COOY has the above mentioned meanings and the corresponding heavy metal salts have at least one repeating unit (II) wherein R₁= R₃ = H and R₂ = CO-(CH₂)₂-COO⁻ and at least one repeating unit (II) wherein R₂ = R₃= H, R₁= CO-(CH₂)₂-COO⁻, X is selected from the group consisting of: silver, gold, copper, zinc, z is comprised between 1 and 3 and p is comprised between 0.6 and 3.

In the process according to the present invention for preparing the succinic acid hemiesters with hyaluronic acid or with hyaluronic acid partial esters, in step (a) the hyaluronic acid is preferably converted to the corresponding pyridinium salt. In particular this conversion encompasses a previous dissolution of the hyaluronate sodium salt in a mixture of water and dimethylformamide, a treatment with a cationic exchange resin for obtaining the corresponding free hyaluronic acid. After removal of the resin the solution is neutralized with pyridine and the pyridinium salt is thus obtained.

In step (b) or (b') of both processes the amount of succinic anhydride is not critical, although it is preferable to add high excess with respect to hyaluronic acid. In fact the best results are obtained when the molar ratio of succinic anhydride /free OH groups present in the repeating unit (III) 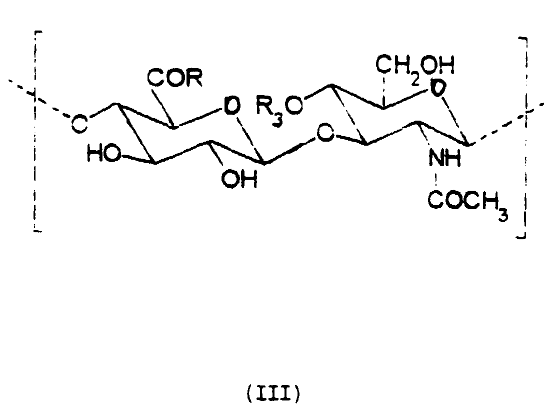 wherein R has the above mentioned meanings,
of the starting hyaluronic acid or hyaluronic acid partial ester, ranges between 15 and 90. Although the temperature is not critical. the best results are obtained if step (b) or (b') of both processes is carried out at 70°C. The preferred organic base used as catalyst in step (b) or (b') of both processes is selected from the group consisting of 4-dimethylaminopyridine , pyridine, or mixtures thereof. By using large amounts of 4-dimethylamminopyridine a succinic acid hemiester with hyaluronic acid or a hyaluronic acid ester with a high degree of succinylation is obtained. by using pyridine alone or in admixture with small quantities of 4-dimethylaminopyridine a succinic acid hemiester with hyaluronic acid with a low degree of succinylation is obtained. Anyway the stronger the reaction conditions, such as temperature, reaction times etc., the greater the degree of esterification of the derivatives formed.

For the preparation of the Ag salt of the succinate hemiester with hyaluronic acid or a hyaluronic acid ester, in step (c) or (c') the succinic acid hemiester with hyaluronic acid or the succinic acid hemiester with hyaluronic acid ester is preferably treated with an aqueous solution of silver nitrate to form the silver salt of succinate hemiester with hyaluronic acid or hyaluronic acid ester.

The Ag salt according to the present invention precipitates from the solution and is recovered by filtration or centrifugation. The precipitate is then washed with ethanol and vacuum dried at 40°C.

The silver compounds of the succinyl derivatives are prepared in the complete dark. All the operations to prepare the silver nitrate solutions, and to prepare the succinyl silver hyaluronate were performed in the dark and the resulting products were stored away from sources of light.

For the preparation of the Cu salts of the succinate hemiester with hyaluronic acid or a hyaluronic acid ester, in step (c) or (c') of both processes, the succinic acid hemiester with hyaluronic acid or the succinic acid hemiester with hyaluronic acid ester is preferably treated with an aqueous solution of CuCl₂ to form the Cu salt of succinate hemiester with hyaluronic acid or with the hyaluronic acid ester.

For the preparation of the Zn salts of the succinate hemiester with hyaluronic acid or a hyaluronic acid ester, in step (c) or (c') of both processes the succinic acid hemiester with hyaluronic acid or the succinic acid hemiester with hyaluronic acid ester is preferably treated with an aqueous solution of ZnCl₂ to form the Zn salts of the succinate hemiester with hyaluronic acid or with the hyaluronic acid ester.

For the preparation of the Au salts of the succinate hemiester with hyaluronic acid or a hyaluronic acid ester, in step (c) or (c') of both processes the succinic acid hemiester with hyaluronic acid or the succinic acid hemiester with hyaluronic acid ester is preferably treated with an aqueous solution of HAuCl₄ to form the Au salts of the succinate hemiester with hyaluronic acid or with the hyaluronic acid ester.

The pharmaceutical compositions according to the present invention to be used for the treatment of burns, wounds and ophthalmia preferably contain the Ag salt according to the present invention and are moreover in the form of ointments, creams gels.

The pharmaceutical compositions to be used for the treatment of osteoarticular diseases preferably contain Au, Zn, Cu salts or mixtures thereof.

We report hereafter some specific examples for the preparation of O-succinylhyaluronates and relative heavy metal salts, but any variation not specifically reported in the following examples is to be considered as coming within the scope of the present invention.

### Example for the preparation of succinic acid hemiester with hyaluronic acid having the repeating unit (I)

### Example 1:

A solution of sodium hyaluronate (HA-Na, 1 g, MW 160,000) in distilled water (35 ml) and N,N-dimethylformamide (DMF, 100 ml) was stirred for ten minutes in the presence of ion exchange resin (3 G, IR 120 H +), after which the resin was removed by filtration after further dilution with DMF (100 ml). The solution was then neutralized with an excess of pyridine (10 ml) to give the pyridine salt of hyaluronic acid (HA-Py). The viscous solution was then carefully evaporated in a vacuum to remove the water present, taking care not to allow the total volume of solution to drop below about 100 ml. This procedure was repeated three times, each time adding DMF (20 ml). The solution was then treated with succinic anhydride (3 g) and pyridine (10 ml) while being stirred at room temperature for 24 hours. The reaction mixture was then concentrated, gathered with distilled water (20 ml), dialized against distilled water (3 times 750 ml) and freeze-dried to give hyaluronic acid succinylate (930 mg).

Table 1 shows the assignment of the chemical shift values of the ¹³C.n.m.r. (50.3 MHz) spectrum of sample 1.

**- TABLE 1 -**

| Chemical shift in δ ppm | non-modified HA | modified HA | other groups |
|---|---|---|---|
| 101.49 | N-1 | | |
| 55.19 | N-2 | | |
| 83.30 | N-3 | | |
| 69.30 | N-4 | | |
| 76.23 | N-5 | | |
| 61.99 | N-6 | | |
| 103.82 | G-1 | | |
| 73.21 | G-2 | | |
| 79.98 | G-3 | | |
| 80.81 | G-4 | | |
| 76.23 | G-5 | | |
| 173.84 | G-6 | | |
| 175.63 | N=C=O | | |
| 102.50 | | N-1 | |
| 83.00 | | N-3 | |
| 73.85 | | N-5 | |
| 64.08 | | N-6 | |
| 71.74 | | G-2 | |
| 29.79, 29.91 | | | CH₂ succinate |
| 175.35. 177.71 | | | C=O succinate |

N.M.R. Analysis shows a degree of succinylation on carbon 6 of the Nacetylglucosamine (N-6) of 0.2 (mol of succinic acid/mol of repeating unit of the polymer).

### Example 2:

A solution of sodium hyaluronate (HA-Na, 1 g, MW 30,000) in distilled water (35 ml) and N,N-dimethylformamide (DMF, 100 ml) was stirred in the presence of ion exchange resin (3 g, IR 120 H+) for 10 minutes and then the resin was removed by filtration after further dilution with DMF (100 ml). The solution was then neutralized with an excess of pyridine (10 ml) to give the pyridine salt of hyaluronic acid (HA-Py). The viscous solution was then carefully evaporated in a vacuum to remove the water present, without allowing the total volume of the solution to drop below about 100 ml. This water-removing procedure was repeated three times, each time with the addition of DMF (20 ml). The solution was then treated with succinic anhydride (3 g) and pyridine (10 ml) while being stirred at 70°C for 24 hours. The reaction mixture was then concentrated, gathered with distilled water (20 ml), dialized against distilled water (3 times 750 ml) and freeze-dried to give hyaluronic acid succinylate (900 mg).

Table 2 reports the assignment of the chemical shift values of the ¹³C:n.m.r. spectrum (50.3 MHz) of sample 2).

**- TABLE 2 -**

| Chemical shift in δ ppm | non-modified HA | modified HA | other groups |
|---|---|---|---|
| 101.77 | N-1 | | |
| 54.33 | N-2 | | |
| 82.91 | N-3 | | |
| 69.93 | N-4 | | |
| 76.31 | N-5 | | |
| 60.95 | N-6 | | |
| 102.77 | G-1 | | |
| 72.58 | G-2 | | |
| 73.88 | G-3 | | |
| 80.94 | G-4 | | |
| 74.13 | G-5 | | |
| 170.00 | G-6 | | |
| 171.83 | N=C=O | | |
| 102.50 | | N-1 | |
| 83.00 | | N-3 | |
| 73.85 | | N-5 | |
| 63.36 | | N-6 | |
| 70.73 | | G-2 | |
| 28.79 | | | CH₂ succinate |
| 168.98, 173.00 | | | C=O succinate |

N.M.R. analysis gives a degree of succinylation on carbon 6 of the Nacetylglucosamine (N-6) of about 0.45 (mol of succinic acid/mol of repeating unit.

### Example 3:

A solution of sodium hyaluronate (HA-Na, 0.5 g, MW 160,000) in distilled water (35 ml) and N,N-dimethylformamide (DMF 100 ml) was stirred in the presence of ion exchange resin (3 G, IR 120 H+) for 10 minutes and then the resin was removed by filtration after further dilution with DMF (75 ml). The solution was then neutralized with an excess of pyridine (6 ml) to give the pyridine salt of hyaluronic acid (HA-Py). the viscous solution was then carefully evaporated in a vacuum to remove the water present, without allowing the total volume of the solution to drop below about 50 ml. This water-removing procedure was repeated three times, each time with the addition of DMF (10 ml). The solution was then treated with succinic anhydride (2 g), 4-dimethylaminopyridine (10 mg) and pyridine (10 ml), while stirring at 70°C for 48 hours. Further quantities of succinic anhydride were added (1 g) and pyridine (2.5 ml) and the mixture was stirred for another 24 hours. The reaction mixture was then concentrated, gathered with distilled water (20 ml), dialized against distilled water (3 times 750 ml) for 3 days and freeze-dried to give hyaluronic acid succinylate (450 mg). The product was characterized by a high degree of viscosity when dissolved in water, the n.m.r. spectrum in particular was characterized by wide peaks due to the sample's high degree of viscosity. The degree of modification was assessed by potentiometric assay, and proved to be 1.8 (mol of succinic acid/mol of repeating unit).

### Example 4:

A solution of sodium hyaluronate (HA-NA, 0.5 g, MW 240,000) in distilled water (60 ml) and N,N-dimethylformamide (DMF 60 ml) was stirred in the presence of ion exchange resin (1 G, IR 120 H+) for 10 minutes, after which the resin was removed by filtration after further dilution with DMF (50 ml). the solution was then neutralized with an excess of pyridine (6 L) to give the pyridine salt of hyaluronic acid (HA-Py). The viscous solution was then carefully evaporated in a vacuum to remove the water present, without allowing the total volume of the solution to drop below about 100 ml. This water-removing procedure was repeated three times, each time with the addition of DMF (20 ml). The gelatin like solution was then treated with succinic anhydride (2 g) and pyridine (5 ml) at 70°C, while being stirred for 18 hours. Further quantities of succinic anhydride (2.5 g) and 4-dimethylaminopyridine (200 mg) were added and the mixture was stirred for another 24 hours. The reaction mixture was then concentrated, gathered with distilled water (20 ml) and freeze-dried to give hyaluronic acid succinylate (450 mg). The product is characterized by being highly viscous when dissolved in water, the n.m.r. spectrum in particular is characterized by very wide peaks due to the highly viscous character of the samples. The degree of modification was assessed by potentiometric assay and the result was 2.5 (mol of succinic acid/mol of repeating unit).

### Example 5:

A solution of sodium hyaluronate (HA-Na, 1 g, MW 40,000) in distilled water (60 ml) and N,N-dimethylformamide (DMF 60 ml) was stirred in the presence of ion exchange resin (1 g, IR 120 H+) for 10 minutes, after which the resin was removed by filtration after further dilution with DMF (50 ml). The solution was then neutralized with an excess of pyridine (10 ml) to give the pyridine salt of hyaluronic acid (HA-Py). The viscous solution was then carefully evaporated in a vacuum to remove the water present, without allowing the total volume of the solution to drop below 50 ml. This water-removing procedure was repeated three times, each time with the addition of DMF (20 ml). The solution was then treated with succinic anhydride (3 g) and pyridine (10 ml) at 70°C while stirring for 18 hours. Further quantities of succinic anhydride (2.5 g) and 4dimethylaminopyridine (200 mg) were added and the mixture was stirred for another 24 hours. The reaction mixture, which was brown in colour, was then concentrated, gathered with distilled water (20 ml), dialized against distilled water (3 times 750 ml) and freeze-dried to give hyaluronic acid succinylate (850 mg). The degree of succinylation was assessed by potentiometric assay and was 3.5 (mol of succinic acid/mol of repeating unit).

### Example 6:

A solution of sodium hyaluronate (HA-Na, 0.5 g. MW 760,000) in distilled water (60 ml) and N,N-dimethylformamide (DMG 60 ml) was stirred in the presence of ion exchange resin (1 g, IR 120 H+) for 10 minutes, after which the resin was removed by filtration after further dilution with DMF (50 ml). The solution was then neutralized with an excess of pyridine (6 ml) to give the pyridine salt of hyaluronic acid (HA-Py). The viscous solution was then carefully evaporated to remove the water present, without allowing the total volume of solution to drop below about 50 ml. This procedure was repeated three times, each time with the addition of DMF (20 ml). The gelatin-like solution was then treated with succinic anhydride (2 g) and 4-dimethylaminopyridine (200 mg) and the mixture was stirred for another 24 hours. The reaction mixture was then concentrated, gathered with distilled water (20 ml), dialized against distilled water (3 times 750 ml) and freeze-dried to give hyaluronic acid succinylate (430 mg). The product is characterized by being highly viscous when dissolved in water, the n.m.r. spectrum in particular is characterized by very wide peaks due to the highly viscous character of the samples. The degree of modification was assessed by potentiometric assay and was 2.5 (mol of succinic acid/mol of repeating unit).

### Examples of the preparation of silver salts of 0-succinyl hyaluronate Example 7:

100 mg of 0-succinyl hyaluronate, prepared as described in Example 1 were dissolved in 10 ml of distilled water. The polymer solution was then supplemented with 10 ml of a solution of AgNO₃ 1N. The white precipitate thus formed was kept in suspension while being stirred constantly for two hours, and was then gathered by filtration through a Buchner funnel, washed several times with ethanol and dried in a vacuum oven set at 40°C. All these operations were performed in the dark to avoid the formation of silver oxide. Atomic absorption analysis showed a silver content of 23.5% in weight. equal to 87% of the theoretical stoichiometric value.

### Example 8:

70 mg of hyaluronic acid succinylate, prepared as described in Example 3 were dissolved in 14 ml of distilled water. The polymer solution, which was highly viscous. was supplemented with 14 ml of a solution of AgNO₃ 1N. A grey precipitate formed immediately and was kept in suspension while being constantly stirred for two hours, after which it was gathered by filtration through a Buchner funnel. It was washed several times with ethanol and dried in a vacuum oven set at 40°C. All these operations were performed in the dark to avoid the formation of silver oxide. Atomic absorption analysis showed the silver content to be 27% in weight, equal to 71% of the theoretical stoichiometric value.

### Example 9:

100 mg of hyaluronic acid succinylate, prepared as described in Example 4, were dissolved in 20 ml of distilled water. The polymer solution, which was highly viscous, was supplemented with 20 ml of a solution of AgNO₃ 2N. A white precipitate formed immediately and was kept in suspension while being constantly stirred for two hours. It was then recovered by filtration through a Buchner funnel, washed several times with ethanol and dried in a vacuum oven set at 40°C. All these operations were performed in the dark to avoid the formation of silver oxide. Atomic absorption analysis showed the silver content to be 28.8% in weight, equal to 70.5% of the theoretical stoichiometric value.

### Example 10:

100 mg of hyaluronic acid succinylate, prepared as described in Example 5, were dissolved in 10 ml of distilled water. The polymer solution, which was highly viscous, was supplemented with 10 ml of a solution of AgNO₃ 1N. A brownish precipitate formed immediately and was kept in suspension while being constantly stirred for two hours, after which it was recovered by filtration through a Buchner funnel, washed several times with ethanol and dried in a vacuum oven at 40°C.

All these operations were performed in the dark to avoid the formation of silver oxide. Atomic absorption analysis showed the silver content to be 31%, equal to 70.2% of the theoretical stoichiometric value.

### Example 11:

100 mg of hyaluronic acid succinylate, prepared as described in Example 6, were dissolved in 10 ml of distilled water. The polymer solution, which was highly viscous, was supplemented with 10 ml of a solution of AgNO₃ 1N. A brownish precipitate was immediately formed, which was kept in suspension while being constantly stirred for two hours, after which it was recovered by filtration through a Buchner funnel, washed several times with ethanol and dried in a vacuum oven set at 40°C. All these operations were performed in the dark to avoid the formation of silver oxide. Atomic absorption analysis showed the silver content to be 27% in weight, equal to 71% of the theoretical stoichiometric value.

### Examples of the preparation of zinc salts of hyaluronic acid succinylate

### Example 12:

100 mg of hyaluronic acid succinylate, prepared as described in Example 1 were dissolved in 10 ml of distilled water. The polymer solution was then supplemented with 10 ml of a solution of ZnCl₂ 0.2 N. The solution was stirred constantly for 2 hours. after which 3 volumes of ethanol were added to precipitate the soluble zinc salt. The precipitate was recovered by centrifugation at 3,000 rpm for 15 minutes, washed several times with ethanol and dried in a vacuum oven set at 40°C. Atomic absorption analysis showed a zinc content of 10%, equal to 101% of the theoretical stoichiometric value.

### Example 13:

100 mg of hyaluronic acid succinylate prepared as described in Example 3 were dissolved in 20 ml of distilled water. The polymer solution, which was highly viscous, was supplemented with 20 ml of a solution of ZnCl₂ 2 N. After the addition of zinc salt, a powdery precipitate was formed, which was recovered by centrifugation at 3,000 rpm for 15 minutes, washed several times with ethanol and dried in a vacuum oven set at 40°C. Atomic absorption analysis showed the zinc content in the sample to be 15.3%, equal to 105% of the theoretical stoichiometric value.

### Example 14:

100 mg of hyaluronic acid succinylate prepared as described in Example 4 were dissolved in 20 ml of distilled water. The polymer solution, which was highly viscous, was supplemented with 20 ml of a solution of ZnCl₂ 2N. After the addition of zinc salt, a powdery precipitate was formed which was recovered by centrifugation at 3,000 rpm for 15 minutes, washed several times with ethanol and dried in a vacuum oven set at 40°C. Atomic absorption analysis showed the zinc content of the sample to be 17.7% in weight, equal to 105% of the theoretical stoichiometric value.

### Example of the preparation of the copper salt of hyaluronic acid succinylate

### Example 15:

100 mg of hyaluronic acid succinylate prepared as described in Example 5 were dissolved in 10 ml of distilled water. The polymer solution was then supplemented with 10 ml of a solution of CuCl₂ 2N. After the addition of copper salt a blue precipitate was formed which was recovered by centrifugation at 3,000 rpm for 15 minutes, washed several times with ethanol and dried in a vacuum oven set at 40°C. Atomic absorption analysis showed the copper content of the sample to be 21.4% in weight, equal to 110% of the theoretical stoichiometric value. It is therefore probable that a small amount of copper salt is incorporated by the polymer during precipitation of the derivative.

### Example of the preparation of gold salt of hyaluronic acid succinylate

### Example 16:

100 mg of hyaluronic acid succinylate prepared as described in Example 3 were dissolved in 20 ml of distilled water. The polymer solution, which was highly viscous, was then supplemented with 20 ml of a solution of HAuCL₄ 0.5N. After addition of gold salt, a precipitate was formed which was recovered by centrifugation at 3,000 rpm for 15 minutes, washed several times with ethanol and dried in a vacuum oven at 40°C. The gold content in the sample proved to be 13% in weight, equal to 44% of the theoretical stoichiometric value.

## Claims

1. Succinic acid hemiester of hyaluronic acid, or of a hyaluronic acid partial or total ester having the following repeating unit (I): 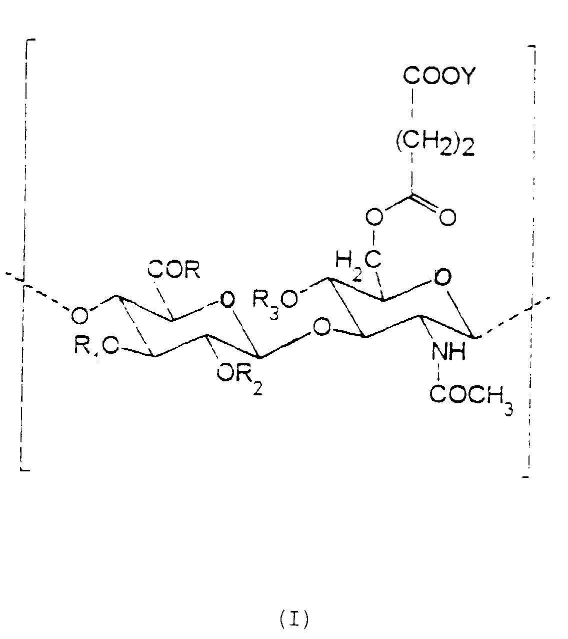 wherein R₁, R₂ and R₃ equal or different from each other are H or CO-(CH₂)₂-COOY, wherein Y is H, R is OH, or an alcoholic residue.

2. The succinic acid hemiester according to claim 1 wherein in the repeating unit (I) R₁ = R₂ = R₃ = H.

3. The succinic acid hemiester according to claim 1 having at least one repeating unit (I) wherein R₁= R₃ = H and R₂ = CO-(CH₂)₂-COOY and at least one repeating unit (I), wherein R₂ = R₃ = H, and R₁ =CO-(CH₂)₂-COOY.

4. Heavy metal salt of succinic acid hemiester with hyaluronic acid or with a hyaluronic acid total or partial ester, having the following repeating unit (II) 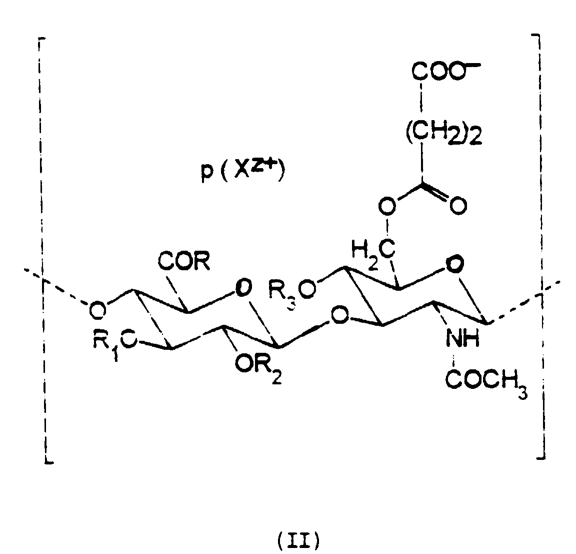 wherein R₁, R₂ and R₃ equal or different from each other are H or CO-(CH₂)₂-COO⁻, R is O⁻, or an alcoholic residue, (X^{z+}) is a cation of a heavy metal in which z is a number comprised between 1 and 6, p is an integer or a decimal number, comprised between 0.1 and 5 provided that p(X^{z+}) is equal to the number of anionic groups COO⁻ present in said repeating unit.

5. The heavy metal salt according to claim 4 wherein X is selected from the group consisting of Ag, Cu. Zn, Au, Ce, W.

6. The heavy metal salt according to one of claims 4 and 5 wherein R₁ = R₂= R₃ = H, X is selected from the group consisting of: silver, gold. copper, zinc, z is comprised between 1 and 3 and p is comprised between 0.3 and 2.

7. The heavy metal salt according to one of claims 4,5 and 6, having at least one repeating unit (II) wherein R₁= R₃ = H and R₂ = CO-(CH₂)₂-COO⁻ and at least one repeating unit (II) wherein R₃ = R₃= H, R₁= CO-(CH₂)₂-COO⁻, X is selected from the group consisting of: silver, gold, copper, zinc, z is comprised between 1 and 3 and p is comprised between 0.6 and 3.

8. A process for preparing the heavy metal salt of succinic acid hemiester with hyaluronic acid or with a partial ester of hyaluronic acid according to one of claims 4, 5, 6 and 7 comprising the following steps:
a) converting the hyaluronic acid sodium salt into a salt selected from the group consisting of pyridinium, tetraalkylammonium, tetraarylammonium, tetraalkylphosphonium, tetraarylphosphonium salt, in the presence of water and an aprotic solvent,
b) treating the solution coming from step (a) with succinic anhydride in the presence of an organic base, as the catalyst, removing the pyridinium tetraalkylammonium, tetraarylammonium, tetraalkylphosphonium, or tetraarylphosphonium cation by dialysis thereby obtaining the succinic acid hemiester with hyaluronic acid or a partial ester thereof having at least one repeating unit (I) 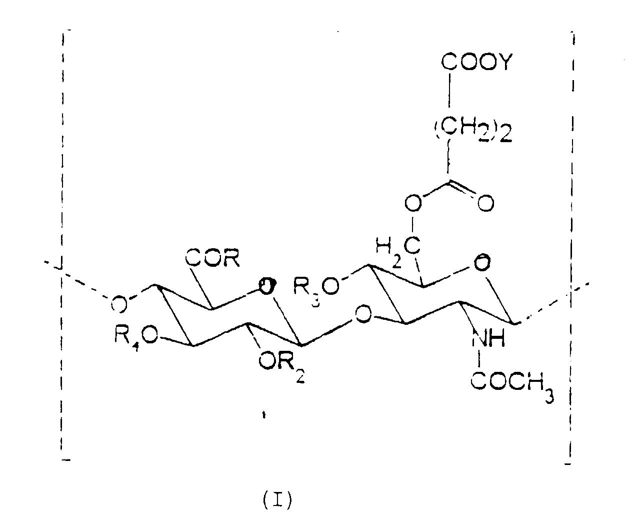 wherein R₁, R₂ and R₃ equal or different from each other are H or CO-(CH₂)₂-COOY, wherein Y is H, R is OH, or an alcoholic residue, provided that at least in one repeating unit (I) R is OH,
and optionally recovering the obtained product by freeze-drying,
c) treating the solution directly coming from the preceding step or an aqueous solution of the recovered solid product coming from the preceding step with an aqueous solution of an inorganic salt of the heavy metal, and recovering the product by filtration and vacuum drying.

9. The process according to claim 8 wherein in step (a) the hyaluronic acid is converted to the corresponding pyridinium salt by using the following operating conditions:
i) dissolving of the hyaluronate sodium salt in a mixture of water and dimethylformamide,
ii) a treatment with a cationic exchange resin to obtain the corresponding free hyaluronic acid.
iii) neutralizing the reaction mixture with pyridine thereby obtaining the pyridinium salt.

10. A process for preparing the heavy metal salts of succinic acid hemiesters with a hyaluronic acid total ester comprising the following steps:
b') treating the hyaluronic acid ester dissolved or suspended in a mixture of water and an aprotic solvent with succinic anhydride in the presence of an organic base, thereby obtaining the succinic acid hemiester having the repeating unit (I) 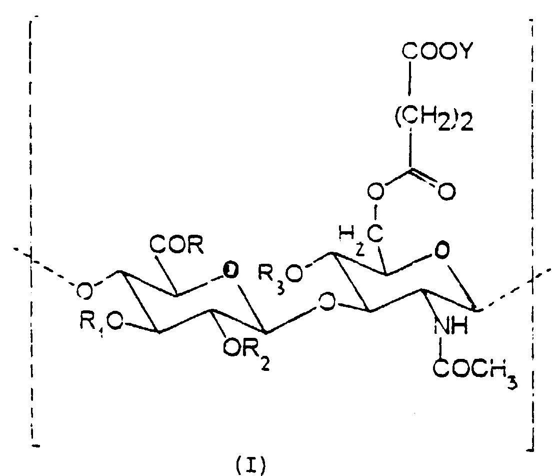 wherein R₁, R₂ and R₃ equal or different from each other are H or CO-(CH₂)₂-COOY, wherein Y is H, R is a residue of an alcohol, and optionally recovering the obtained product by freeze-drying,
c') treating the solution directly coming from the preceding step or an aqueous solution of the recovered solid product coming from the preceding step with an aqueous solution of an inorganic salts of the heavy metal, and recovering the product by filtration and vacuum drying.

11. The process according to one of claims 8 and 9 or the process according to claim 10 wherein step (b) or (b') is carried out by using a molar ratio of succinic anhydride /free OH groups present in the following repeating unit (III), 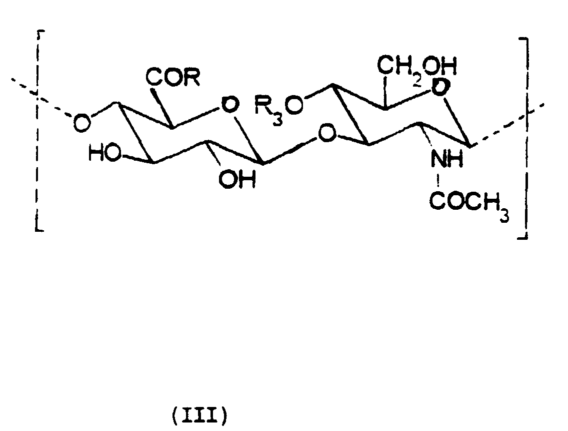 of the starting hyaluronic acid or hyaluronic acid partial or total ester, which ranges between 15 and 90 and at 70°C and the catalyst is selected from the group consisting of 4-dimethylaminopyridine , pyridine, or mixtures thereof.

12. The process according to one of claims 8. 9 or 11 or the process according to one of claims and 10 and 11 for preparing the Ag salts of the succinic acic hemiester with hyaluronic acid or a hyaluronic acid ester wherein in step (c) or (c') the succinic acid hemiester with hyaluronic acid or the succinic acid hemiester with a hyaluronic acid total or partial ester is treated with an aqueous solution of silver nitrate.

13. The process according to one of claims 8, 9 or 11 or the process according to one of claims 10 and 11 for preparing the Zn salts of the succinic acid hemiester with hyaluronic acid or with a hyaluronic acid ester wherein in step (c) or (c') the succinic acid hemiester with hyaluronic acid or with hyaluronic acid total or partial ester is treated with an aqueous solution of ZnCl₂.

14. The process according to one of claims 8, 9 and 11 or the process according to one of claims 10 and 11 for preparing the Cu salts of the succinic acic hemiester with hyaluronic acid or a hyaluronic acid ester wherein in step (c) or (c') the succinic acid hemiester with hyaluronic acid or the succinic acid hemiester with hyaluronic acid ester is treated with an aqueous solution of CuCl₂.

15. The process according to one of claims 8, 9 and 11 or the process according to one of claims 10 and 11 for preparing the Au salts of the succinic acid hemiester with hyaluronic acid or a hyaluronic acid ester wherein in step (c) or (c') the succinic acid hemiester with hyaluronic acid or the succinic acid hemiester with hyaluronic acid ester is treated with an aqueous solution of HAuCl₄.

16. A therapeutic composition containing as the active ingredient at least one heavy metal salt of succinic acid hemiester with hyaluronic acid or a hyaluronic acid total or partial ester according to one of claims 4, 5, 6, or 7 optionally in association with other active ingredients having a similar therapeutical activity.

17. The therapeutic composition according to claim 16 for the treatment of burns, wounds and ophthalmia.

18. The therapeutic compositions according to one of claims 16 or 17 in the form of ointments, creams gels.

19. The therapeutic compositions according to one of claims 16, 17 or 18 containing as the active ingredients an Ag salt of a succinic acid hemiester with hyaluronic acid or a hyaluronic acid total or partial ester.

20. The therapeutic compositions according to claim 16 for the treatment of osteoarticular diseases.

21. The therapeutic compositions according to one of claims 16 and 20, containing as the active ingredient at least one heavy metal salt of succinic acid hemiester with hyaluronic acid or with a hyaluronic acid ester selected from the group consisting of salts of Au, Cu, and Zn, or mixtures thereof.

22. A health care composition containing at least one heavy metal salt of succinic acid hemiester with hyaluronic acid or a hyaluronic acid ester according to one of claims 4, 5, 6 or 7.

23. Biomaterial containing at least one heavy metal salt according to one of claims 4, 5. 6 or 7.

## Patentansprüche

1. Bernsteinsäurehalbester von Hyaluronsäure oder eines Hyaluronsäurepartial oder -vollesters, der die folgende wiederkehrende Einheit (I) aufweist 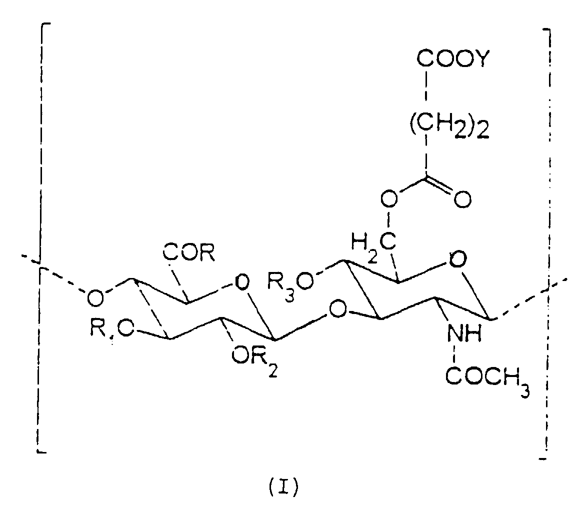 worin bedeuten:
R₁, R₂ und R₃, die gleich oder voneinander verschieden sind, H oder CO-(CH₂)₂-COOY,
Y H und
R OH oder einen alkoholischen Rest.

2. Bernsteinsäurehalbester nach Anspruch 1, bei dem in der wiederkehrenden Einheit (I) R₁ = R₂ = R₃ = H.

3. Bernsteinsäurehalbester nach Anspruch 1, der aufweist mindestens eine wiederkehrende Einheit (I), in der R₁ = R₃ = H und R₂ = CO-(CH₂)₂-COOY, und mindestens eine wiederkehrende Einheit (I), in der R₂ = R₃ = H, und R₁ = CO-(CH₂)₂-COOY.

4. Schwermetallsalz des Bernsteinsäurehalbesters mit Hyalorunsäure oder einem Hyaluronsäurepartial- oder -vollester, das die folaende wiederkehrende Einheit (II) aufweist: 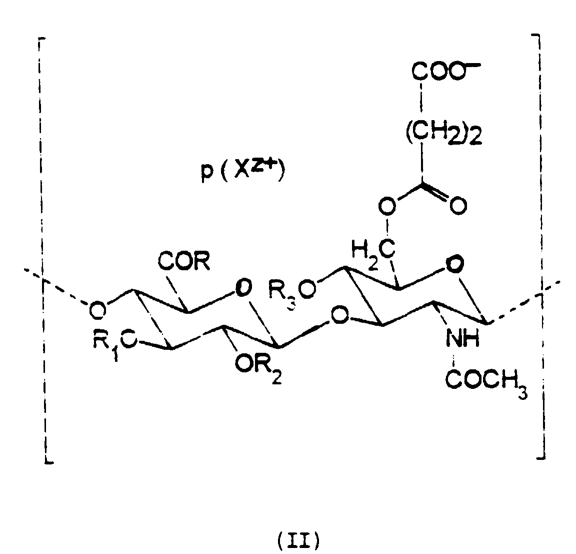 worin bedeuten:
R₁, R₂ und R₃, die gleich oder voneinander verschieden sind, H oder CO-(CH₂)₂-COO⁻,
R O⁻ oder einen alkoholischen Rest,
(X^{z+}) ein Kation eines Schwermetalls, in dem z eine Zahl zwischen 1 und 6 darstellt, und
p eine ganze Zahl oder eine Dezimalzahl zwischen 0,1 und 5,
mit der Maßgabe, daß p(X^{z+}) gleich der Anzahl der in der wiederkehrenden Einheit vorhandenen anionischen Gruppen COO⁻ ist.

5. Schwermetallsalz nach Anspruch 4, worin X ausgewählt wird aus der Gruppe, die besteht aus Ag, Cu, Zn, Au, Ce und W.

6. Schwermetallsalz nach einem der Ansprüche 4 und 5, worin R₁ = R₂ = R₃ = H, X ausgewählt wird aus der Gruppe, die besteht aus Silber, Gold, Kupfer, Zink, z zwischen 1 und 3 liegt und p zwischen 0,3 und 2 liegt.

7. Schwermetallsalz nach einem der Ansprüche 4, 5 und 6, das aufweist mindestens eine wiederkehrende Einheit (II), worin R₁ = R₃ = H und R₂ = CO-(CH₂)₂-COO⁻, und
mindestens eine wiederkehrende Einheit (II), worin R₂ = R₃ = H, R₁ = CO-(CH₂)₂-COO⁻,
X ausgewählt wird aus der Gruppe, die besteht aus Silber, Gold, Kupfer, Zink, z zwischen 1 und 3 liegt und p zwischen 0,6 und 3 liegt.

8. Verfahren zur Herstellung des Schwermetallsalzes des Bernsteinsäurehalbesters mit Hyaluronsäure oder einem Partialester der Hyaluronsäure nach einem der Ansprüche 4, 5, 6 und 7, das die folgenden Stufen umfaßt:
a) Umwandlung des Hyaluronsäure-Natriumsalzes in ein Salz, ausgewählt aus der Gruppe, die besteht aus Pyridinium-, Tetraalkylammonium-, Tetraarylammonium-, Tetraalkylphosphosphonium- und Tetraarylphosphoniumsalzen, in Gegenwart von Wasser und eines aprotischen Lösungsmittels,
b) Behandeln der in der Stufe (a) erhaltenen Lösung mit Bernsteinsäureanhydrid in Gegenwart einer organischen Base als Katalysator, Entfernen des Pyridinium-, Tetraalkylammonium-, Tetraarylammonium-, Tetraalkylphosphonium- oder Tetraarylphosphonium-Kations durch Dialyse unter Bildung des Bernsteinsäurehalbesters mit Hyaluronsäure oder einem Partialester derselben, der mindestens eine wiederkehrende Einheit (I) aufweist 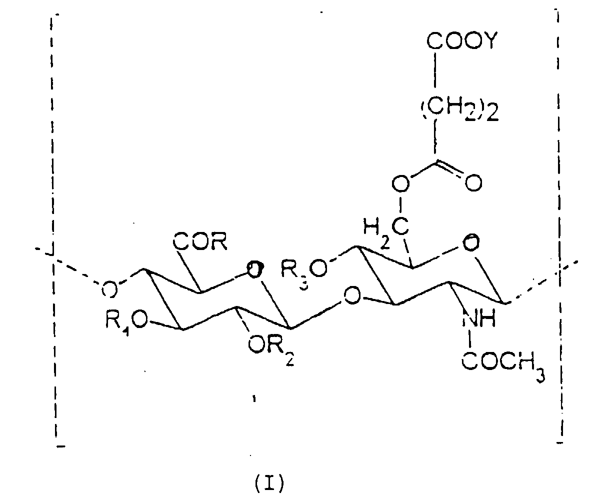 worin R₁, R₂ und R₃, die gleich oder voneinander verschieden sind, für H oder CO-(CH₂)₂-COOY; Y für H und R für OH oder einen alkoholischen Rest stehen, mit der Maßgabe, daß mindestens in einer wiederkehrenden Einheit (I) R OH bedeutet, und gegebenenfalls Abtrennung (Gewinnung) des erhaltenen Produkts durch Gefriertrocknen und
c) Behandeln der direkt aus der vorhergehenden Stufe stammenden Lösung oder einer wäßrigen Lösung des abgetrennten festen Produkts aus der vorhergehenden Stufe mit einer wäßrigen Lösung eines anorganischen Salzes des Schwermetalls und Abtrennung (Gewinnung) des Produkts durch Filtration und Vakuumtrocknen.

9. Verfahren nach Anspruch 8, bei dem in der Stufe (a) die Hyaluronsäure durch Anwendung der folgenden Arbeits-Bedingungen in das entsprechende Pyridiniumsalz überführt wird:
i) Auflösen des Hyaluronat-Natriumsalzes in einem Gemisch von Wasser und Dimethylformamid,
ii) Behandeln mit einem Kationenaustauscherharz zur Herstellung der entsprechenden freien Hyaluronsäure und
iii) Neutralisieren der Reaktionsmischung mit Pyridin zur Herstellung des Pyridiniumsalzes.

10. Verfahren zur Herstellung der Schwermetallsalze von Bernsteinsäurehalbestern mit einem Hyaluronsäurevollester, das die folgenden Stufen umfaßt:
b') Behandeln des Hyaluronsäureesters, der in einem Gemisch von Wasser und einem aprotischen Lösungsmittel gelöst oder suspendiert ist, mit Bernsteinsäureanhydrid in Gegenwart einer organischen Base zur Herstellung des Bernsteinsäurehalbesters, der die wiederkehrende Einheit (I) aufweist 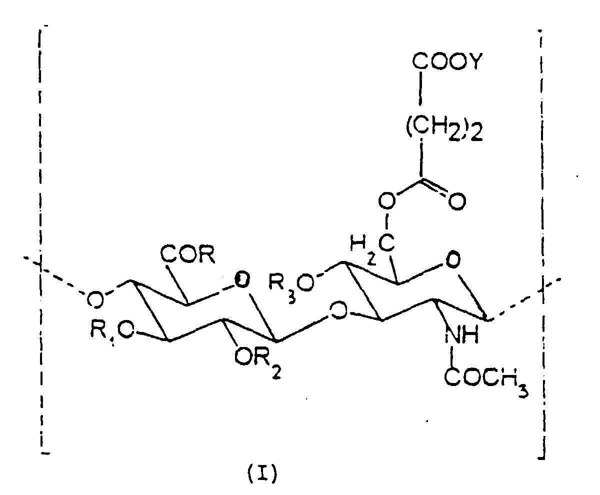 worin R₁, R₂ und R₃, die gleich oder voneinander verschieden sind, für Wasserstoff oder CO-(CH₂)₂-COOY stehen; Y für H steht und R für einen Rest eines Alkohols steht, und gegebenenfalls Abtrennen (Gewinnen) des erhaltenen Produkts durch Gefriertrocknen, und
c') Behandeln der Lösung, die direkt aus der vorhergehenden Stufe stammt, oder einer wäßrigen Lösung des abgetrennten festen Produkts, das aus der vorhergehenden Stufe stammt, mit einer wäßrigen Lösung eines anorganischen Schwermetallsalzes und Abtrennen des Produkts durch Filtration und Vakuumtrocknen.

11. Verfahren nach einem der Ansprüche 8 und 9 oder nach Anspruch 10, bei dem die Stufe (b) oder (b') durchgeführt wird durch Anwendung eines Molverhältnisses Bernsteinsäureanhydrid/freie OH-Gruppen, die in der wiederkehrenden Einheit (III) vorliegen 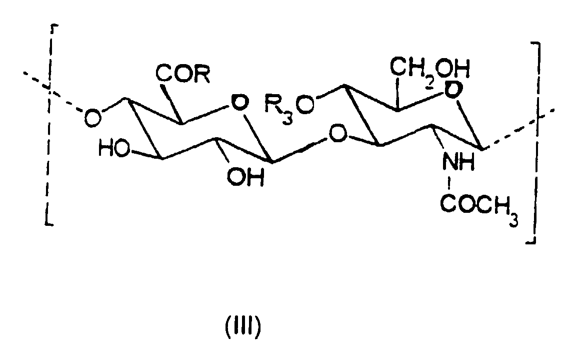 der Ausgangs-Hyaluronsäure oder des Hyaluronsäurepartial- oder -vollesters, das in dem Bereich zwischen 15 und 90 liegt, und bei einer Temperatur von 70°C und unter Verwendung eines Katalysators, der ausgewählt wird aus der Gruppe, die besteht aus 4-Dimethylaminopyridin, Pyridin oder Mischungen davon.

12. Verfahren nach einem der Ansprüche 8, 9 oder 11 oder nach einem der Ansprüche 10 und 11 zur Herstellung der Ag-Salze des Bernsteinsäurehalbesters mit Hyaluronsäure oder einem Hyaluronsäureester, bei dem in der Stufe (c) oder (c') der Bernsteinsäurehalbester mit Hyaluronsäure oder der Bernsteinsäurehalbester mit einem Hyaluronsäurepartial- oder -vollester mit einer wäßrigen Silbernitratlösung behandelt wird.

13. Verfahren nach einem der Ansprüche 8, 9 oder 11 oder nach einem der Ansprüche 10 und 11 zur Herstellung der Zn-Salze des Bernsteinsäurehalbesters mit Hyaluronsäure oder einem Hyaluronsäureester, bei dem in der Stufe (c) oder (c') der Bernsteinsäurehalbester mit Hyaluronsäure oder einem Hyaluronsäurepartial- oder -vollester mit einer wäßrigen ZnCl₂-Lösung behandelt wird.

14. Verfahren nach einem der Ansprüche 8, 9 und 11 oder nach einem der Ansprüche 10 und 11 zur Herstellung der Cu-Salze des Bernsteinsäurehalbesters mit Hyaluronsäure oder einem Hyaluronsäureester, bei dem in der Stufe (c) oder (c') der Bernsteinsäurehalbester mit Hyaluronsäure oder der Bernsteinsäurehalbester mit einem Hyaluronsäureester mit einer wäßrigen CuCl₂-Lösung behandelt wird.

15. Verfahren nach einem der Ansprüche 8, 9 und 11 oder nach einem der Ansprüche 10 und 11 zur Herstellung der Au-Salze des Bernsteinsäurehalbesters mit Hyaluronsäure oder einem Hyaluronsäureester, bei dem in der Stufe (c) oder (c') der Bernsteinsäurehalbester mit Hyaluronsäure oder der Bernsteinsäurehalbester mit einem Hyaluronsäureester mit einer wäßrigen HAuCl₄-Lösung behandelt wird.

16. Therapeutische Zusammensetzung, die als Wirkstoff (aktiven Bestandteil) mindestens ein Schwermetallsalz des Bernsteinsäurehalbesters mit Hyaluronsäure oder einem Hyaluronsäurepartial- oder -vollester nach einem der Ansprüche 4, 5, 6 oder 7, gegebenenfalls in Assoziation mit anderen Wirkstoffen (aktiven Bestandteilen) mit einer ähnlichen therapeutischen Aktivität, enthält.

17. Therapeutische Zusammensetzung nach Anspruch 16 zur Behandlung von Verbrennungen, Wunden und Ophthalmien.

18. Therapeutische Zusammensetzungen nach einem der Ansprüche 16 oder 17 in Form von Salben, Cremes oder Gelen.

19. Therapeutische Zusammensetzungen nach einem der Ansprüche 16, 17 oder 18, die als Wirkstoffe (aktive Bestandteile) ein Ag-Salz eines Bernsteinsäurehalbesters mit Hyaluronsäure oder einem Hyaluronsäurepartial- oder -vollester enthalten.

20. Therapeutische Zusammensetzungen nach Anspruch 16 zur Behandlung von Knochengelenks-Erkrankungen.

21. Therapeutische Zusammensetzungen nach einem der Ansprüche 16 und 20, die als Wirkstoff (aktiven Bestandteil) mindestens ein Schwermetallsalz des Bernsteinsäurehalbesters mit Hyaluronsäure oder einem Hyaluronsäureester enthalten, das ausgewählt wird aus der Gruppe, die besteht aus Au-, Cu- und Zn-Salzen oder Mischungen davon.

22. Gesundheitspflege- bzw. Hygiene-Zusammensetzung, die mindestens ein Schwermetallsalz des Bernsteinsäurehalbesters mit Hyaluronsäure oder einem Hyaluronsäureester nach einem der Ansprüche 4, 5, 6 oder 7 enthält.

23. Biologisches Material, das mindestens ein Schwermetallsalz nach einem der Ansprüche 4, 5, 6 oder 7 enthält.

## Revendications

1. Semi-ester de l'acide succinique avec de l'acide hyaluronique ou avec un ester partiel ou total de l'acide hyaluronique, ayant le motif répété (I) : 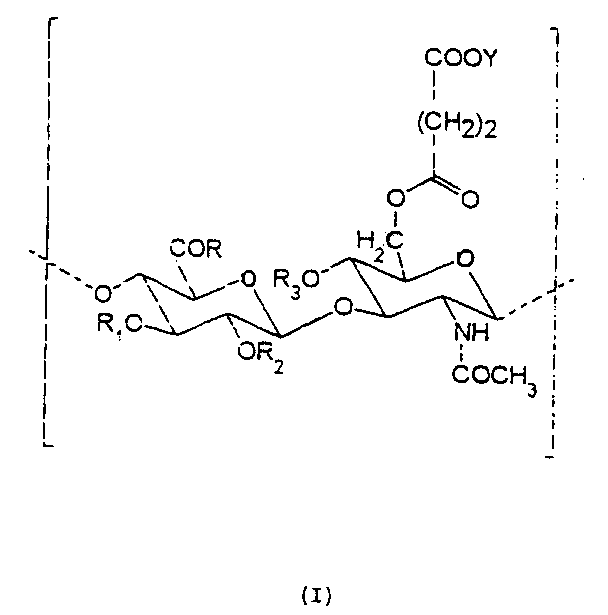 dans lequel R₁, R₂ et R₃, égaux ou différents entre eux, sont H ou CO-(CH₂)₂-COOY, où Y est H, R est OH, ou un résidu alcoolique.

2. Semi-ester de l'acide succinique selon la revendication 1, dans lequel, dans le motif répété (I), R₁=R₂=R₃=H.

3. Semi-ester de l'acide succinique selon la revendication 1, ayant au moins un motif répété (I) dans lequel R₁=R₃=H et R₂=CO-(CH₂)₂-COOY, et au moins un motif répété (I) dans lequel R₂=R₃=H et R₁=CO-(CH₂)₂-COOY.

4. Sel de métal lourd d'un semi-ester de l'acide succinique avec de l'acide hyaluronique ou avec un ester total ou partiel de l'acide hyaluronique, ayant le motif répété (II) suivant 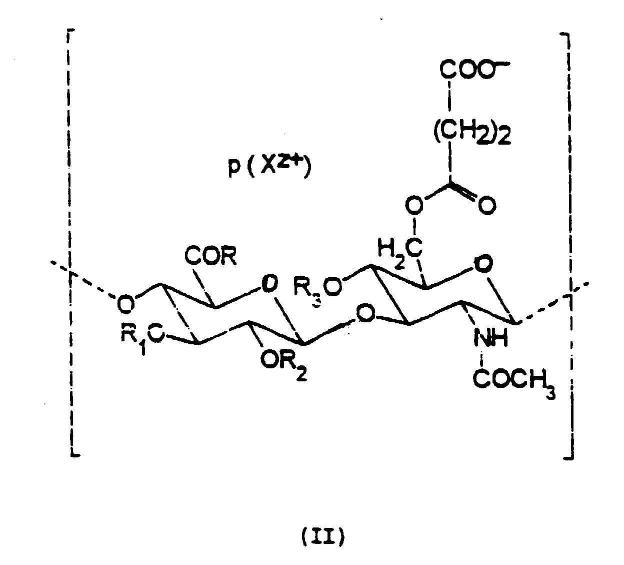 dans lequel R₁, R₂ et R₃, égaux ou différents entre eux, sont H ou CO-(CH₂)₂-COO⁻, R est O⁻ ou un résidu alcoolique, (X^{z+}) est un cation d'un métal lourd dans lequel z est un nombre compris entre 1 et 6, p est un entier ou un nombre décimal, compris entre 0,1 et 5, étant entendu que p(X^{z+}) est égal au nombre de groupes anioniques COO⁻ présents dans ledit motif répété.

5. Sel de métal lourd selon la revendication 4, dans lequel X est choisi dans le groupe constitué par Ag, Cu, Zn, Au, Ce, W.

6. Sel de métal lourd selon l'une des revendications 4 et 5, dans lequel R₁ = R₂ = R₃ =H, X est choisi dans le groupe constitué par : l'argent, l'or, le cuivre, le zinc, z est compris entre 1 et 3, et p est compris entre 0,3 et 2.

7. Sel de métal lourd selon l'une des revendications 4, 5 et 6, ayant au moins un motif répété (II) dans lequel R₁=R₃=H et R₂=CO-(CH₂)₂-COO⁻ et au moins un motif répété (II) dans lequel R₂=R₃=H, R₁= CO-(CH₂)₂-COO⁻, X est choisi dans le groupe constitué par : l'argent, l'or, le cuivre, le zinc, z est compris entre 1 et 3 et p est compris entre 0,6 et 3.

8. Procédé pour préparer le sel de métal lourd d'un semi-ester d'acide succinique avec de l'acide hyaluronique ou avec un ester partiel de l'acide hyaluronique selon l'une des revendications 4, 5, 6 et 7, comprenant les étapes suivantes consistant :
a) à convertir le sel sodique de l'acide hyaluronique en un sel choisi dans le groupe constitué par le sel de pyridinium, de tétraalkylammonium, de tétraarylammonium, de tétraalkylphosphonium, de tétraarylphosphonium, en présence d'eau et d'un solvant aprotique,
b) à traiter la solution provenant de l'étape (a) avec de l'anhydride succinique en présence d'une base organique, en tant que catalyseur, à éliminer le cation pyridinium, tétraalkylammonium, tétraarylammonium, tétraalkylphosphonium ou tétraarylphosphonium par dialyse, de façon à obtenir le semi-ester d'acide succinique avec de l'acide hyaluronique ou un ester partiel de celui-ci ayant au moins un motif répété (I) 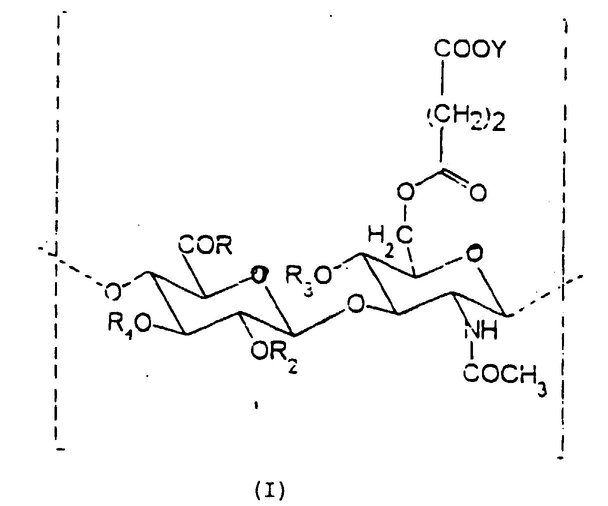 dans lequel R₁, R₂ et R₃, égaux ou différents entre eux, sont H ou CO-(CH₂)₂-COOY, où Y est H, R est OH, ou un résidu alcoolique, étant entendu qu'au moins dans un motif répété (I) R est OH, et à récupérer facultativement le produit obtenu par lyophilisation,
c) à traiter la solution provenant directement de l'étape précédente ou une solution aqueuse du produit solide récupéré provenant de l'étape précédente avec une solution aqueuse d'un sel minéral du métal lourd, et à récupérer le produit par filtration et séchage sous vide.

9. Procédé selon la revendication 8, dans lequel, dans l'étape (a), l'acide hyaluronique est converti en le sel de pyridinium correspondant en utilisant les conditions opératoires suivantes consistant :
i) à dissoudre le sel sodique hyaluronate dans un mélange d'eau et de diméthylformamide,
ii) à traiter avec une résine échangeuse de cations pour obtenir l'acide hyaluronique libre correspondant,
iii) à neutraliser le mélange réactionnel avec de la pyridine, de façon à obtenir le sel de pyridinium.

10. Procédé pour préparer les sels de métal lourd de semi-esters de l'acide succinique avec un ester total de l'acide hyaluronique, comprenant les étapes suivantes consistant :
b') à traiter l'ester de l'acide hyaluronique dissous ou en suspension dans un mélange d'eau et d'un solvant aprotique avec de l'anhydride succinique en présence d'une base organique, de façon à obtenir le semi-ester de l'acide succinique ayant le motif répété (I) 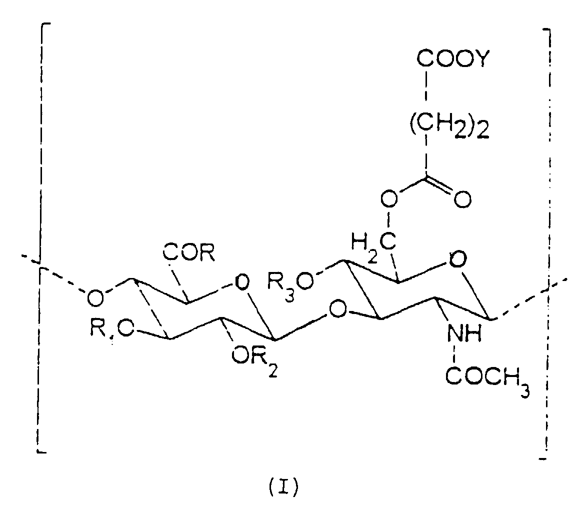 dans lequel R₁, R₂ et R₃, égaux ou différents entre eux, sont H ou CO-(CH₂)₂-COOY, où Y est H, R est un résidu d'un alcool, et à récupérer facultativement le produit obtenu par lyophilisation,
c') à traiter la solution provenant directement de l'étape précédente ou une solution aqueuse du produit solide récupéré provenant de l'étape précédente avec une solution aqueuse d'un sel minéral de métal lourd, et à récupérer le produit par filtration et séchage sous vide.

11. Procédé selon l'une des revendications 8 et 9 ou procédé selon la revendication 10, dans lequel l'étape (b) ou (b') est réalisée en utilisant un rapport molaire anhydride succinique/groupes OH libres présents dans le motif repété (III) suivant, 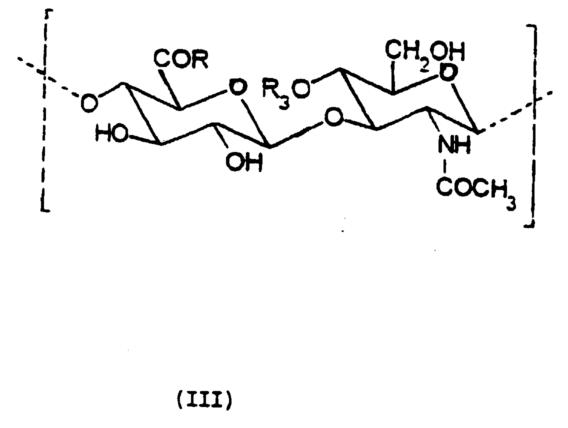 de l'acide hyaluronique ou l'ester partiel ou total de l'acide hyaluronique de départ, qui est entre 15 et 90 et à 70 °C, et le catalyseur est choisi dans le groupe constitué par la 4-diméthylaminopyridine, la pyridine ou des mélanges de celles-ci.

12. Procédé selon l'une des revendications 8, 9 ou 11, ou procédé selon l'une des revendications 10 et 11 pour préparer les sels de Ag du semi-ester de l'acide succinique avec de l'acide hyaluronique ou un ester de l'acide hyaluronique, dans lequel, dans l'étape (c) ou (c'), le semi-ester de l'acide succinique avec de l'acide hyaluronique ou le semi-ester de l'acide succinique avec un ester total ou partiel de l'acide hyaluronique est traité avec une solution aqueuse de nitrate d'argent.

13. Procédé selon l'une des revendications 8, 9 ou 11, ou procédé selon l'une des revendications 10 et 11, pour préparer les sels de Zn du semi-ester de l'acide succinique avec de l'acide hyaluronique ou avec un ester de l'acide hyaluronique, dans lequel, dans l'étape (c) ou (c'), le semi-ester de l'acide succinique avec de l'acide hyaluronique ou avec un ester total ou partiel de l'acide hyaluronique est traité avec une solution aqueuse de ZnCl₂.

14. Procédé selon l'une des revendications 8, 9 et 11, ou procédé selon l'une des revendications 10 et 11, pour préparer les sels de Cu du semi-ester de l'acide succinique avec de l'acide hyaluronique ou un ester de l'acide hyaluronique, dans lequel, dans l'étape (c) ou (c'), le semi-ester de l'acide succinique avec de l'acide hyaluronique ou le semi-ester de l'acide succinique avec un ester de l'acide hyaluronique est traité avec une solution aqueuse de CuCl₂.

15. Procédé selon l'une des revendications 8, 9 et 11, ou procédé selon l'une des revendications 10 et 11, pour préparer les sels de Au du semi-ester de l'acide succinique avec de l'acide hyaluronique ou un ester de l'acide hyaluronique, dans lequel, dans l'étape (c) ou (c'), le semi-ester de l'acide succinique avec de l'acide hyaluronique ou le semi-ester de l'acide succinique avec un ester de l'acide hyaluronique est traité avec une solution aqueuse de HAuCl₄.

16. Composition thérapeutique contenant, en tant qu'ingrédient actif, au moins un sel de métal lourd d'un semi-ester de l'acide succinique avec de l'acide hyaluronique ou un ester total ou partiel de l'acide hyaluronique selon l'une des revendications 4, 5, 6 et 7, facultativement en association avec d'autres ingrédients actifs ayant une activité thérapeutique semblable.

17. Composition thérapeutique selon la revendication 16 pour le traitement de brûlures, de blessures et d'une ophtalmie.

18. Compositions thérapeutiques selon l'une des revendications 16 et 17, sous la forme de pommades, de crèmes gels.

19. Compositions thérapeutiques selon l'une des revendications 16, 17 et 18, contenant en tant qu'ingrédients actifs un sel d'Ag d'un semi-ester de l'acide succinique avec de l'acide hyaluronique ou un ester total ou partiel de l'acide hyaluronique.

20. Compositions thérapeutiques selon la revendication 16 pour le traitement de maladies ostéo-articulaires.

21. Compositions thérapeutiques selon l'une des revendications 16 et 20, contenant en tant qu'ingrédient actif au moins un sel de métal lourd d'un semi-ester de l'acide succinique avec de l'acide hyaluronique ou avec un ester de l'acide hyaluronique choisi dans le groupe constitué par les sels de Au, Cu et Zn, ou des mélanges de ceux-ci.

22. Composition de soins de santé contenant au moins un sel de métal lourd d'un semi-ester de l'acide succinique avec de l'acide hyaluronique ou un ester de l'acide hyaluronique selon l'une des revendications 4, 5, 6 et 7.

23. Biomatière contenant au moins un sel de métal lourd selon l'une des revendications 4, 5, 6 et 7.
